# EUROPÄISCHE PATENTSCHRIFT

(11) **EP 3 938 008 B1**
(45) Veröffentlichungstag und Bekanntmachung des Hinweises auf die Patenterteilung: **04.11.2026**
(21) Anmeldenummer: 20712491.8
(22) Anmeldetag: 13.03.2020
(51) Int. Cl.: A61M 1/36

(54) **BEHANDLUNGSVORRICHTUNG ZUR EXTRAKORPORALEN, IMMUNTOLERANZ-STEIGERNDEN BLUTBEHANDLUNG**
TREATMENT DEVICE FOR AN EXTRACORPOREAL TREATMENT OF BLOOD FOR INCREASING IMMUNOTOLERANCE
DISPOSITIF DU TRAITEMENT POUR TRAITER DU SANG EXTRACORPORELLEMENT POUR AUGMENTER L'IMMUNOTOLERANCE

(30) Priorität: 15.03.2019 DE 102019106651
(43) Veröffentlichungstag der Anmeldung: 19.01.2022
(73) Patentinhaber: Fraunhofer-Gesellschaft zur Förderung der angewandten Forschung e.V., 80686 München (DE)
(72) Erfinder: ZIMMERMANN, Heiko, 66280 Sulzbach (DE); NEUBAUER, Julia, 97082 Würzburg (DE); FISCHER, Benjamin, 97082 Würzburg (DE)
(74) Vertreter: v. Bezold & Partner Patentanwälte - PartG mbB
(86) Internationale Anmeldenummer: PCT/EP2020/056855
(87) Internationale Veröffentlichungsnummer: WO 2020/187741

(56) Entgegenhaltungen:
- EP-B2- 0 260 280
- US-A1- 2017 239 413

## Beschreibung

Die Erfindung betrifft eine Behandlungsvorrichtung, die zur extrakorporalen Blutbehandlung von Blut eines Probanden für eine Immuntoleranz-Steigerung des Probanden eingerichtet ist, ein Kit zur extrakorporalen Blutbehandlung umfassend die Blutbehandlungsvorrichtung und ein in vitro Verfahren zum Betreiben der Behandlungsvorrichtung zur Immuntoleranz-Steigerung durch eine extrakorporale Blutbehandlung. Anwendungen der Erfindung sind in der Medizin und Biochemie, insbesondere bei der Steigerung der Immuntoleranz von Organismen gegenüber Fremd- oder Eigenimplantaten oder bei Autoimmunerkrankungen gegeben.

In der vorliegenden Beschreibung wird auf den folgenden Stand der Technik verwiesen, der den technischen Hintergrund der Erfindung veranschaulicht.
[1] M. Zouali "Immunological Tolerance: Mechanisms", in: Encyclopedia of Life Sciences. John Wiley & Sons, Ltd, Chichester. 2001;
[2] E. Shaban et al. in "Kidney Dis." 4, 205-213, 2018;
[3] DE 10 2017 210 134 A1;
[4] US 4 685 900;
[5] US 2017/0239413 A1; und
[6] EP 0 260 280 B2.

Das Auftreten einer Immunreaktion (Immunantwort) als Reaktion des Immunsystems eines Organismus auf körperfremde oder auch körpereigene Substanzen (Antigene) ist allgemein bekannt. Das Ausbleiben oder die Verringerung einer Immunreaktion auf Antigene wird als Immuntoleranz bezeichnet (siehe z. B. [1]). Wenn eine Immunreaktion Schäden am Organismus verursacht, wird üblicherweise eine immunsuppressive Behandlung (Immunsuppression) unter Verwendung von Medikamenten und/oder Bestrahlungen durchgeführt. Damit sollen beispielsweise Abstoßungsreaktionen nach allogenen Transplantationen, insbesondere Organtransplantationen, vermieden werden. Immunsuppressive Behandlungen haben jedoch aufgrund von Nebenwirkungen erhebliche Nachteile für den behandelten Organismus, wie z. B. das erhöhte Risiko schwer kontrollierbarer Infektionen, eine erhöhte Gefahr, an Krebs zu erkranken, und eine allgemeine Verschlechterung der Lebensqualität.

Es besteht daher ein Interesse an der Vermeidung oder Verringerung von immunsuppressiven Behandlungen. Hierfür wird bisher vor einer allogenen Transplantation gezielt nach Spendermaterial gesucht, das aufgrund der in diesem auftretenden Antigene an das Immunsystem des Empfängers angepasst ist und eine geringe oder fehlende Immunreaktion erwarten lässt. Eine derartige Suche bietet jedoch wegen der beschränkten Anzahl von verfügbaren Spenderorganen oder beim Auftreten von Autoimmunerkrankungen nur einen begrenzten Gewinn bei der Bemühung um eine Minimierung der Immunsuppression.

Des Weiteren ist die Anwendung von Immuntherapien bei Krebserkrankungen bekannt. Bei einer extrakorporalen Immunmodulation wird Blut aus dem erkrankten Organismus entnommen und bei einer Behandlung außerhalb des Körpers modifiziert. Bei der Behandlung werden T-Zellen im Blut so modifiziert, dass sie verstärkt in der Lage sind, Krebszellen anzugreifen. Nach Rückführung des behandelten Blutes in den Organismus ist die Empfindlichkeit des Immunsystems gegenüber den Krebszellen erhöht, so dass die körpereigenen Kräfte zur Bekämpfung der Krebserkrankung verstärkt sind. Die Immunmodulation hat sich durch die Verstärkung von Immunreaktionen als erfolgversprechend bei der Krebsbehandlung erwiesen. Die Immunmodulation ist jedoch für die Unterdrückung von Immunreaktionen oder eine Steigerung der Immuntoleranz, z. B. bei Transplantationen, ungeeignet.

Aus der Transplantationsmedizin ist bekannt, dass Immunreaktionen im Zeitverlauf nach einer allogenen Transplantation abnehmen, so dass bei günstigem Verlauf die immunsuppressive Behandlung beendet werden kann. Des Weiteren ist bekannt, dass sich eine hohe Antigenkonzentration günstig auf die Entwicklung einer gesteigerten Immuntoleranz auswirkt [1] und dass Wirkstoffe, wie z. B. der Wirkstoff Rapamycin [2], die Entwicklung der Immuntoleranz fördern können.

Es ist auch bekannt, zur Behandlung von Allergien spezifische Immuntherapien durchzuführen (Hyposensibilisierung), wobei dem erkrankten Organismus körperfremde Allergene zugeführt werden, um die Immuntoleranz zu erhöhen. Derartige Immuntherapien haben jedoch, insbesondere wegen der Einführung der Allergene in den Organismus, auch den Nachteil eines hohen Nebenwirkungsrisikos.

Aus [3] ist ein System zur extrakorporalen Blutbehandlung mit drei Blutbehandlungsvorrichtungen bekannt, die eine Adsorbervorrichtung und/oder eine Plasmatrennvorrichtung, eine Dialysevorrichtung und eine Gasaustauschvorrichtung umfassen. Die Adsorbervorrichtung kann zur Adsorption z. B. von Antikörpern ausgebildet sein. Die Anwendung dieses System ist ähnlich wie andere Techniken zur Blutwäsche auf die Modifizierung von Blut z. B. bei Stoffwechselerkrankungen beschränkt. Auch in [4] wird ein System zur extrakorporalen Blutbehandlung beschrieben, bei dem Biopolymere, die mit biologisch wirksamen Substanzen ausgestattet sind, dem Blut pathologische Komponenten entziehen. Eine Steigerung der Immuntoleranz ist mit den Systemen gemäß [3] oder [4] nicht möglich.

Aus [5] ist die Behandlung von Zellen durch eine Wechselwirkung mit biologisch wirksamen Molekülen auf einem mikrostrukturierten Substrat bekannt. In [6] wird ein Verfahren zur Selektion vorbestimmter Targetzellen aus einer Probe durch eine Wechselwirkung mit einer Wirksubstanz beschrieben.

Die Aufgabe der Erfindung ist es, eine verbesserte Behandlungsvorrichtung zur extrakorporalen Blutbehandlung, ein verbessertes Kit zur extrakorporalen Blutbehandlung und/oder ein verbessertes Verfahren zur extrakorporalen Blutbehandlung bereitzustellen, mit denen Nachteile herkömmlicher Techniken vermieden werden und die insbesondere eine Steigerung der Immuntoleranz eines Probanden, eine Verminderung oder den Ausschluss von Nebenwirkungen der Blutbehandlung, ein vermindertes Risiko von Abstoßungsreaktionen und/oder eine schonende Behandlung von Autoimmunkrankheiten ermöglichen.

Diese Aufgaben werden jeweils entsprechend durch eine Behandlungsvorrichtung zur extrakorporalen Blutbehandlung, ein Kit zur extrakorporalen Blutbehandlung und ein Verfahren zur extrakorporalen Blutbehandlung gelöst, welche die Merkmale der unabhängigen Ansprüche aufweisen. Bevorzugte Ausführungsformen und Anwendungen der Erfindung werden in den abhängigen Ansprüchen definiert.

Gemäß einem ersten allgemeinen Gesichtspunkt der Erfindung wird die obige Aufgabe durch eine Behandlungsvorrichtung gelöst, die zur extrakorporalen Blutbehandlung von Blut eines Probanden für eine Immuntoleranz-Steigerung des Probanden eingerichtet ist, umfassend
- eine Zellexpositionseinrichtung, die biologisches Zellmaterial mit Oberflächenmolekülen aufweist, das für eine stoffliche Wechselwirkung mit der Blutprobe, umfassend eine Reaktion von körpereigenen Immunzellen im Blut mit den Oberflächenmolekülen des Zellmaterials, im Innenraum des Behälters angeordnet ist, gekennzeichnet durch
- mindestens einen Behälter, der ein nach außen verschließbares Gefäß ist und einen Innenraum zur Aufnahme einer Blutprobe vom Blut des Probanden und eine Austauscheinrichtung aufweist, die zur Zuleitung und/oder Ableitung der Blutprobe (1) in oder aus dem Innenraum des Behälters eingerichtet ist, wobei
- die Zellexpositionseinrichtung im Behälter positioniert ist, wobei
- das biologische Zellmaterial zelläquivalente iPS-Zellen und/oder -Zellbestandteile, welche die gleichen Antigene tragen wie Zellen, aus denen die iPS-Zellen gewonnen wurden, und/ oder organäquivalent differenzierte iPS-Zellen und/oder -Zellbestandteile, welche die gleichen Antigene tragen wie Zellen der Organe, aus denen die iPS-Zellen gewonnen wurden, umfasst. Die extrakorporale Blutbehandlung ist eine Veränderung einer Blutprobe des Bluts des Probanden in der Behandlungsvorrichtung außerhalb des Körpers (Organismus) des Probanden. Der Proband kann ein menschlicher Organismus (insbesondere Patient, Transplantatempfänger) oder ein tierischer Organismus (Säugetierorganismus), z. B. zur Evaluierung einer Zelltherapie bei einem Tierversuch sein. Die Behandlungsvorrichtung umfasst mindestens einen Behälter (auch als Kartusche bezeichnet) mit starren und/oder biegsamen Behälterwänden, wobei der Behälter einen Innenraum (auch als Reaktionskammer bezeichnet) zur Aufnahme einer Blutprobe des Probanden aufweist. Der Innenraum hat ein Volumen z. B. im Bereich von 1 ml bis 1 l. Der Behälter ist erfindungsgemäß ein nach außen verschließbares, insbesondere steril verschließbares Gefäß, in dem die Blutprobe während der extrakorporalen Behandlung ruhend oder bewegt angeordnet ist. Des Weiteren ist der Behälter mit einer Austauscheinrichtung ausgestattet, die zum Blutaustausch mit dem Organismus, insbesondere zur Zuleitung und/oder Ableitung der Blutprobe in oder aus dem Innenraum des Behälters, eingerichtet ist. Der Blutaustausch mit dem Organismus kann ein direkter Austausch, bei dem die Behandlungsvorrichtung direkt mit dem Blutkreislauf des Probanden verbunden ist, oder ein mittelbarer Austausch sein, bei dem die Behandlungsvorrichtung mit einem gesonderten Zufuhr- und/oder Auffangreservoir verbunden ist.

Gemäß der Erfindung weist die Behandlungsvorrichtung eine Zellexpositionseinrichtung auf. Die Zellexpositionseinrichtung weist biologisches Zellmaterial auf, das im Behälter zu dessen Innenraum hin freiliegend so angeordnet ist, dass die Blutprobe im Innenraum mit dem biologischen Zellmaterial stofflich wechselwirken kann. Die Zellexpositionseinrichtung ist im Behälter angeordnet, d. h. innerhalb der Behälterwände positioniert oder in eine der Behälterwände integriert. Das biologische Zellmaterial ist mit der Zellexpositionseinrichtung lokal beschränkt in einem Zellmaterialbereich angeordnet, der von dem Innenraum zumindest lokal räumlich abgegrenzt ist. Die Zellexpositionseinrichtung ist so konfiguriert, dass das Blut mit dem Zellmaterial oder Oberflächenmolekülen, z. B. Antigenen, von diesem in Kontakt kommt, insbesondere Blutzellen Oberflächen des Zellmaterials oder Oberflächenmoleküle des Zellmaterials berühren, ohne dass Zellen des Zellmaterials in den Innenraum eintreten. Die stoffliche Wechselwirkung zwischen dem Blut und dem Zellmaterial erfolgt durch eine gegenseitige Berührung an der Grenzfläche zwischen dem Innenraum und dem Zellmaterialbereich oder durch einen Eintritt von Oberflächenmolekülen des Zellmaterials in den Innenraum.

Die Zellexpositionseinrichtung stellt eine Exposition mit dem Zellmaterial bereit, dem die Blutprobe im Innenraum ausgesetzt wird. Vorteilhafterweise kommt es zu Blutprobe-Zellmaterial-Wechselwirkungen, insbesondere zu einer Reaktion der körpereigenen Immunzellen in der Blutprobe mit den Oberflächen-Antigenen des präsentierten Zellmaterials, die eine Immunmodulation bewirkt, sich aber wegen der extrakorporalen Behandlung nicht unmittelbar auf den Probanden auswirkt. Das Blut wird außerhalb des Körpers an das Zellmaterial (insbesondere Antigene des Zellmaterials) angepasst (adaptive Immunabwehr) und erwirbt dadurch eine gesteigerte Immuntoleranz. Durch Rückführung des behandelten Blutes in den Probanden wird die gesteigerte Immuntoleranz auf den Probanden übertragen. Die Immuntoleranz des Probanden wird unmittelbar durch die behandelte Blutprobe und/oder durch eine Interaktion der behandelten Blutprobe, insbesondere von durch die Behandlung aktivierten T-Zellen, mit dem Immunsystem des Probanden (z.B. in den Lymphknoten) etabliert. Durch eine einmalige oder mehrmalige Behandlung von Blut des Probanden wird dessen Immuntoleranz gesteigert, so dass Transplantate mit einem geringeren oder verschwindenden Risiko von Abstoßungsreaktionen eingeführt werden können oder Autoimmunerkrankungen abgemildert oder geheilt werden können.

Die Behandlungsvorrichtung ist vorzugsweise ein Einwegartikel, der einfach oder mehrfach ausschließlich bei einem einzigen Probanden angewendet und nach Anwendung entsorgt wird.

Gemäß einem zweiten allgemeinen Gesichtspunkt der Erfindung wird die obige Aufgabe durch ein Kit zur extrakorporalen Blutbehandlung gelöst, umfassend mindestens eine Behandlungsvorrichtung gemäß dem ersten allgemeinen Gesichtspunkt der Erfindung, wobei die Austauscheinrichtung für eine unmittelbare Verwendung an einem Behandlungsort, wie z. B. in einer Arztpraxis oder einer Klinik, konfiguriert ist. Hierzu weist die Austauscheinrichtung vorzugsweise mindestens einen Schlauch und mindestens eine sterile Injektionsnadel auf. Im Kit zur extrakorporalen Blutbehandlung kann die mindestens eine Behandlungsvorrichtung oder mindestens die Zellexpositionseinrichtung der mindestens einen Behandlungsvorrichtung im gefrorenen Zustand vorgesehen sein. Wenn die Behandlungsvorrichtung, insbesondere die Zellexpositionseinrichtung mit dem biologischen Zellmaterial, im eingefrorenen Zustand bereitgestellt wird, erfolgt vor der Einleitung der Blutprobe in den Behälter ein Auftauen der Behandlungsvorrichtung.

Gemäß einem dritten allgemeinen Gesichtspunkt der Erfindung wird die obige Aufgabe durch ein Verfahren zum Betreiben der Behandlungsvorrichtung gemäß dem ersten allgemeinen Gesichtspunkt der Erfindung gelöst. Eine zu behandelnde Blutprobe vom Blut eines Probanden wird bereitgestellt und in den Behälter der Behandlungsvorrichtung gefüllt. Die Blutprobe im Behälter wird der Behandlung durch eine Wechselwirkung der Blutprobe, insbesondere der Immunzellen der Blutprobe, mit dem biologischen Zellmaterial unterzogen. Die Dauer der Behandlung wird vorzugsweise im Bereich von 5 Minuten bis 8 Stunden gewählt. Nach der Behandlung zeichnet sich die behandelte Blutprobe durch eine gesteigerte Immuntoleranz, d. h. eine verringerte Immunreaktion gegenüber dem Zellmaterial, aus. Abschließend erfolgt eine Entnahme der Blutprobe aus dem Behälter.

Der Begriff "Blutprobe" bezieht sich auf ein vorbestimmtes Volumen des Bluts des Probanden, wie es durch eine Entnahme aus dem Blutkreislauf und ggf. eine Zwischenlagerung in einem Reservoir, bereitgestellt wird, oder auf eine Suspension von zellulären Bestandteilen, insbesondere Immunzellen, des Bluts des Probanden. Im letzteren Fall kann die Suspension z. B. nach der Entnahme von Blut aus dem Blutkreislauf durch eine Auftrennung des Bluts und eine Entfernung von Bestandteilen, die sich nicht auf die Immunreaktion auswirken, z. B. Blutplasma oder andere Bestandteile, gewonnen werden. Für die Steigerung der Immuntoleranz wird vorzugsweise eine Blutprobe mit einem Gesamtvolumen im Bereich von 1 ml bis 1 l behandelt.

Die Erfindung bietet die folgenden wesentlichen Vorteile. Erstmalig wird eine Immuntoleranz über das Blut ausschließlich unter Verwendung einer Selbstregulation des Probanden, insbesondere eines Transplantatempfängers, induziert. Im Gegensatz zu herkömmlichen Immuntherapien, wie z. B. der Hyposensibilisierung bei Allergien, findet erfindungsgemäß die Blut-Zellmaterial-Interaktion außerhalb des Körpers in der Behandlungsvorrichtung, insbesondere in dem Behälter, statt. Weiterhin werden im Gegensatz zu herkömmlichen Therapieansätzen zur Förderung einer Immuntoleranz keine viralen Vektoren (direkt oder indirekt) in den Probanden eingebracht. Es wird kein körperfremdes Material oder nur eine so extrem geringe Mengen davon in den Körper des Probanden rücküberführt, so dass massive Nebenwirkungen, wie z.B. ein anaphylaktischer Schock ausgeschlossen sind.

Vorteilhafterweise erfolgt eine gezielte Verminderung der Immunreaktion und der Vermittlung der Immuntoleranz in einem Transplantat- Empfänger (Proband). Hierbei wird eine Immunreaktion auf biologisches, z. B. körperfremdes Zellmaterial (z.B. Zellen, Zellbestandteile, Zellmischungen, Gewebe oder ganze Organe) durch einfache oder wiederholte Exposition von einer oder mehreren Blutproben des Probanden mit dem z. B. körperfremden Zellmaterial vermindert. Da Immunreaktionen auf körperfremdes Material oft drastische Immunreaktionen im Empfänger verursachen, werden die im Blut befindlichen Teile des Immunsystems, dem körperfremden biologischen Material in der Behandlungsvorrichtung ausgesetzt und nicht im Probanden.

Das Verfahren der Erfindung ist ein *in vitro* Verfahren zum Betreiben der Behandlungsvorrichtung der Erfindung zur extrakorporalen Blutbehandlung von Blut eines Probanden für eine Immuntoleranz-Steigerung des Probanden, umfassend die Schritte:
- Bereitstellen einer zu behandelnden Blutprobe vom Blut des Probanden in einem Zufuhrreservoir,
- Einleitung der Blutprobe von dem Zufuhrreservoir in den Behälter der Behandlungsvorrichtung,
- Behandlung der Blutprobe durch eine Wechselwirkung, umfassend eine Reaktion der körpereigenen Immunzellen mit den Oberflächenmolekülen des präsentierten Zellmaterials derart, dass die behandelte Blutprobe geeignet ist, eine gesteigerte Immuntoleranz des Probanden zu bewirken, und
- Ausleitung der Blutprobe aus dem Behälter in ein Auffangreservoir, wobei
- die Blutbehandlung erfolgt, ohne dass der Proband anwesend ist.

Ein Verfahren zur Verminderung der Immunreaktion des Probanden auf das biologische Zellmaterial umfasst die folgenden Schritte. Zunächst wird dem Probanden, vorzugsweise vor einer Transplantation von körperfremden Zellmaterial, wiederholt Blut entnommen und in die Behandlungsvorrichtung überführt (dieser Schritt ist kein Teil der Erfindung). In der Behandlungsvorrichtung befinden sich an den Innenraum angrenzend Bestandteile des zu transplantierenden körperfremden Biomaterials oder das körperfremde Biomaterial selbst, welches von dem zu transplantierenden Material/ Gewebe/ Organ/ Spender stammt oder dieses nachahmt. Dies wird z. B. durch induziert pluripotente Stammzellen oder aus von diesen Stammzellen abgeleiteten Zellen realisiert. Somit kann der Empfänger schon vor der eigentlichen Transplantation immunologisch an das Transplantat gewöhnt werden.

Während die Blutprobe in dem Innenraum des Behälters inkubiert wird, reagieren die immunkompetenten Bestandteile des Blutes mit den präsentierten Antigenen des Zellmaterials. Dabei kann die Menge des körperfremden Zellmaterials an die Verwendung oder die Immunreaktion des Probanden angepasst werden. Somit können in dem Behälter lokal hohe Antigenkonzentrationen erreicht werden, welche als besonders förderlich für die Entwicklung einer Immuntoleranz angesehen sind (siehe [1]). Aber auch geringe Antigenkonzentrationen können realisiert werden, in dem weniger körperfremdes Zellmaterial in dem Behälter enthalten ist. Im Rahmen einer Therapie zur Entwicklung einer Immuntoleranz können auch mehrere Behälter mit unterschiedlichen Mengen körperfremden Zellmaterials zum Einsatz kommen.

Nach der Inkubation wird die Blutprobe, ggf. nach einer Aufbereitung, wieder in den Probanden überführt (dieser Schritt ist kein Teil der Erfindung), und dabei werden auch die immunkompetenten Bestandteile der Blutprobe zurück in den Probanden überführt. Die Gedächtnisfunktion und die regulatorische Wirkung der immunkompetenten Bestandteile (T- und B- Zellen, regulatorische T-Zellen, T-Gedächtniszellen, Makrophagen, natürliche Killerzellen und dendritische Zellen) wird somit in den Körper zurückgeführt und kann dort mit dem Immunsystem des Probanden interagieren und/oder dessen Immuntoleranz beeinflussen.

Durch die wiederholte Exposition und Rückführung in den Patienten wird so ein immunmodulierender Effekt induziert, welcher vorteilhafterweise zu einer Immuntoleranz schon vor der Transplantation führt. Von besonderem Vorteil ist, dass die Immunreaktion außerhalb des Körpers des Probanden stattfindet, jedoch mit biologischem Zellmaterial des Spenders durchgeführt werden kann.

Wenn gemäß einer bevorzugten Ausführungsform der Erfindung die Zellexpositionseinrichtung eine Trennschicht aufweist, mit der das biologische Zellmaterial vom Innenraum getrennt ist, können sich Vorteile für die Unterdrückung einer Einführung des Zellmaterials oder größerer Bestandteile von diesem, wie Zellorganellen, Membranstücke oder dergleichen, in die Blutprobe ergeben. Mit der Trennschicht, die eine teildurchlässige Membran oder ein teildurchlässiges Gitternetz umfasst, wird das biologische Zellmaterial an der Zellexpositionseinrichtung gehalten, die Trennschicht für einen Substanzaustausch zwischen dem biologischen Zellmaterial und der Blutprobe eingerichtet ist.

Gemäß einer bevorzugten Variante der Erfindung (im Folgenden: erste Ausführungsform) weist die Zellexpositionseinrichtung einen Träger (Substrat) auf, an dem das biologische Zellmaterial in einem adhärenten Zustand angeordnet ist. Vorteilhafterweise ist das trägergebundene Zellmaterial bei der Vorbereitung der Zellexpositionseinrichtung, z. B. in einem gesonderten Inkubator, und/oder bei der Anordnung im Behälter einfach handhabbar. Ein weiterer Vorteil besteht in der inhärenten Fixierung des Zellmaterials, so dass der vom Innenraum räumlich abgegrenzte Zellmaterialbereich durch die Oberfläche des Zellmaterials auf dem Träger oder die Oberfläche einer Trennschicht auf dem trägergebundenen Zellmaterial gebildet wird. Der Träger mit dem Zellmaterial kann unmittelbar im Innenraum des Behälters angeordnet sind. Das Material, wie z. B. Hydrogel, Kunststoff, Glas oder Keramik, die Struktur, wie z. B. elastisch, oberflächenstrukturiert, kompakt, porös und/oder aus Fasern zusammengesetzt, und die Form des Trägers können aus einer Vielzahl von Varianten gewählt werden. Beispielsweise kann ein flexibler Träger für eine Deformation im Innenraum und somit für eine Bewegung der Blutprobe relativ zum Zellmaterial und/oder für eine Oberflächenvergrößerung durch z.B. Faltung, um mehr biologisches Zellmaterial zu präsentieren, ausgelegt sein. Des Weiteren kann ein Träger eine Vielzahl von Teilsubstraten umfassen, die jeweils das Zellmaterial tragen und mit gegenseitigen Abständen angeordnet sind, so dass eine große innere Oberfläche des Zellmaterials im Innenraum des Behälters bereitgestellt wird. Des Weiteren kann ein Träger ein poröses Material mit einer inneren Oberfläche, welche das Zellmaterial trägt, und einer Porosität umfassen, die einen freien Durchfluss der Blutprobe im Innenraum durch das poröse Material erlaubt. In diesem Fall kann die optional vorgesehene Trennschicht ausschließlich auf der äußeren Oberfläche des porösen Materials angeordnet sein.

Vorteilhafterweise kann der Träger mit einer Bindungsschicht ausgestattet sein, die eine adhärenten Bindung von induzierten pluripotenten Stammzellen (iPS-Zellen), adulten Stammzellen und/oder daraus differenzierten Zellen fördert. Die Bindungsschicht bietet den Vorteil einer verbesserten Fixierung des Zellmaterials am Träger. Insbesondere bei Bereitstellung der Bindungsschicht kann auf die optional vorgesehene Trennschicht der Zellexpositionseinrichtung verzichtet werden.

Gemäß einer alternativen Variante der Erfindung (im Folgenden: zweite Ausführungsform) weist die Zellexpositionseinrichtung alternativ oder zusätzlich zum Träger einen vom Innenraum getrennten Suspensionsraum auf, in dem das biologische Zellmaterial in einem suspendierten Zustand und/oder auf Träger-Beads (Trägerkügelchen, Micro-Carrier) angeordnet ist. Zwischen dem Innenraum und dem Suspensionsraum ist vorzugsweise die Trennschicht der Zellexpositionseinrichtung vorgesehen. Die zweite Ausführungsform bietet den Vorteil einer Bewegung des biologischen Zellmaterials bei gleichzeitiger Trennung von Zellmaterial und der Blutprobe im Innenraum des Behälters.

Ein weiterer wesentlicher Vorteil der Erfindung besteht in der Vielzahl biologischer Zellmaterialien, die zur Behandlung des Blutes des Probanden verfügbar sind. Das biologische Zellmaterial kann menschlichen Ursprungs von einem körperfremden Spender oder vom Probanden selbst sein. Alternativ oder zusätzlich kann das biologische Zellmaterial tierischen Ursprungs sein, z. B. um die Immuntoleranz des Probanden für eine Xeno-Tranplantation (Transplantation von tierischem Material in den Menschen oder umgekehrt) zu steigern oder um Blut eines tierischen Probanden insbesondere für Versuchszwecke zu behandeln. Das biologische Zellmaterial kann intakte Zellen und/oder Zellbestandteile, wie z. B. Zellmembranen umfassen. Das biologische Zellmaterial umfasst zelläquivalente iPS-Zellen und/oder -Zellbestandteile, welche die gleichen Antigene tragen wie Zellen, aus denen die iPS-Zellen gewonnen wurden, und/ oder organäquivalent differenzierte iPS-Zellen und/oder -Zellbestandteile, welche die gleichen Antigene tragen wie Zellen der Organe, aus denen die iPS-Zellen gewonnen wurden.

Gemäß weiteren Alternativen, die nicht Teil der Erfindung sind, kann das biologische Zellmaterial körperfremdes biologisches Zellmaterial organäquivalent differenzierte adulte Stammzellen oder deren Zellbestandteile, biologische Zellen und/oder Zellbestandteilen, welche Antigen-Antikörper-Reaktionen im Blut auslösen, körperfremde Zellen oder Organteile, z. B. Gewebe, wie sie für eine allogene Transplantation in den Probanden vorgesehen sind, und/oder deren Bestandteile, eine Zusammensetzung von Zellmaterial, das verschiedene Zelltypen enthält, welche charakteristische HL-Antigene einer vorbestimmten Referenzgruppe von Probanden, insbesondere einer Bevölkerungsgruppe, tragen, und Zellen, die genetisch für einen von außen und/oder zeitlich stimulierbaren, z. B. Licht- oder Wirkstoffinduzierten, Zelltod modifiziert sind, umfassen.

Zusätzlich kann das Zellmaterial Wirkstoffe umfassen, die eine Immuntoleranzsteigernde Wirkung besitzen oder eine unterstützende Funktion bei der Induktion der Immuntoleranz ausüben. Beispielsweise kann eine Verstärkung der Reaktion des Blutes, eine Verlängerung der Verweildauer von spezifischen Blutbestandteilen, eine Anreicherung von bestimmten Blutbestandteilen, eine Verlängerung der Immuntoleranz, eine Hemmung der Blutgerinnung, eine Verstärkung der Immuntoleranz und/oder eine Vermittlung der Immuntoleranz nach Rückgabe des modulierten Blutes (nachgeschaltete Verstärkung) vorgesehen sein. Das Zellmaterial kann insbesondere Proteine, biologisch-aktive Wirkstoffe, wie z. B. Rapamycin, RNA, DANN und/oder Viren umfassen.

Von besonderem Vorteil ist die gemäß der Erfindung vorgesehene Verwendung zelläquivalenten iPS-Zellen und/oder -Zellbestandteilen (d. h. iPS-Zellen oder -Zellbestandteile, welche die gleichen Antigene tragen wie die Zellen, aus denen die iPS-Zellen gewonnen wurden), organäquivalent differenzierten iPS-Zellen und/oder Zellbestandteilen (d. h. differenzierte iPS-Zellen oder - Zellbestandteile, welche die gleichen Antigene tragen wie die Organe, aus denen die iPS-Zellen gewonnen wurden), da diese die gleichen Antigene wie die entsprechenden Zellen oder Organe des Spenderorganismus tragen und so für die gewünschte Interaktion mit der Blutprobe des Probanden in einem begrenzten Raum, wie dem Innenraum des Behälters, geeignet sind.

Wenn das Zellmaterial eine Zusammensetzung von verschiedenen Zelltypen enthält, welche charakteristische HL-Antigene z. B. einer Bevölkerungsgruppe tragen, kann erfindungsgemäß eine Steigerung der Immuntoleranz erzielt werden, die mit großer Wahrscheinlichkeit für frei wählbare Transplantate von Spendern aus dieser Bevölkerungsgruppe wirksam ist. Vorteilhaftweise wird damit die Behandlung unabhängig vom Vorliegen des konkret vorgesehen Spendermaterials ermöglicht. Selbst wenn mit der Zusammensetzung von verschiedenen Zelltypen nicht genau die Antigene eines Transplantats getroffen werden, ist eine wirksame Steigerung der Immuntoleranz möglich.

Die Verwendung von Zellen, die genetisch für einen von außen und/oder zeitlich stimulierbaren, z. B. Licht- oder Wirkstoffinduzierten, Zelltod modifiziert sind, hat den Vorteil, dass ggf. unbeabsichtigt in den Probanden gelangendes Zellmaterial durch Licht oder Wirkstoffe oder Zeitablauf unwirksam gemacht werden kann.

Die Behandlungsvorrichtung kann des Weiteren dazu eingerichtet sein, bei Autoimmunerkrankungen eine Reduktion der Immunreaktion zu forcieren.

Ein weiterer besonderer Vorteil der Erfindung ist es, dass die Menge und/oder Konzentration des biologischen Zellmaterials in der Behandlungsvorrichtung in Abhängigkeit von einer gegebenen oder erreichten Immuntoleranz des Probanden einstellbar ist. Die Zellexpositionseinrichtung wird so mit dem biologischen Zellmaterial beladen, dass die Steigerung der Immuntoleranz des Probanden optimiert wird.

Gemäß einer weiteren vorteilhaften Ausführungsform der Erfindung weist die Austauscheinrichtung eine Eingangsleitung und eine Ausgangsleitung auf, die in einer fluidischen Verbindung mit dem Innenraum und mit einem gegenseitigen Abstand angeordnet sind. Vorteilhafterweise kann damit der Behälter als Durchflusssystem konfiguriert werden. Besonders bevorzugt sind die Eingangs- und Ausgangsleitungen an zueinander gegenüberliegenden Seiten des Behälters angeordnet. Besonders bevorzugt weisen die Eingangsleitung und die Ausgangsleitung jeweils ein Absperrelement, wie z. B. ein Ventil, auf. Alternativ oder zusätzlich kann die Austauscheinrichtung mit einer Kanüle ausgestattet sein, die zur direkten Verbindung mit dem Blutkreislauf des Probanden eingerichtet ist. Vorteilhafterweise wird damit die Blutbehandlung direkt in Verbindung mit dem Körper des Probanden erleichtert. Alternativ oder zusätzlich kann die Austauscheinrichtung mit einer Pumpe ausgestattet sein, die zum Bluttransport in den oder aus dem Behälter angeordnet ist. Vorteilhafterweise wird damit die Bewegung der Blutprobe unterstützt.

Gemäß einer weiteren bevorzugten Ausführungsform der Erfindung ist die Behandlungsvorrichtung mit einer Rückhalteeinrichtung ausgestattet, die für eine Zurückhaltung des körperfremden biologischen Zellmaterials im Behälter angeordnet ist. Die Rückhalteeinrichtung bietet den Vorteil, dass ein Transport von Teilen des biologischen Zellmaterials, die möglicherweise unbeabsichtigt von der Zellexpositionseinrichtung in den Innenraum überführt wurden, in den Probanden minimiert oder vollständig ausgeschlossen wird. Besonders bevorzugte umfasst die Rückhalteeinrichtung einen mechanisch wirksamen Filter (Filtermaterial, das ausschließlich für Zellen des Zellmaterials undurchlässig ist) oder einen chemisch wirksamen Filter (Oberfläche, an der ausschließlich das Zellmaterial spezifisch bindet). Vorteilhafterweise kann die Rückhalteeinrichtung Teil der Austauscheinrichtung sein, z. B. an einer Ausgangsseite der Austauscheinrichtung angeordnet sein.

Gemäß einer weiteren vorteilhaften Ausführungsform der Erfindung weist die Behandlungsvorrichtung eine Konvektionseinrichtung auf, die zur Bewegung der Blutprobe im Innenraum des Behälters eingerichtet ist. Vorzugsweise wird die Blutprobe während der Behandlung in der Behandlungsvorrichtung bewegt. Die Konvektionseinrichtung bietet den Vorteil, durch die Bewegung der Blutprobe die Wirksamkeit der stofflichen Interaktion der Blutprobe mit dem biologischen Zellmaterial zu vergrößern. Die Konvektionseinrichtung kann z. B. ein Rührwerk im Innenraum und/oder ein Schwenkwerk umfassen, mit dem der Behälter gekoppelt ist. Wenn der Behälter mindestens eine flexible Behälterwand aufweist, kann die Konvektionseinrichtung alternativ oder zusätzlich eine Deformationseinrichtung umfasst, mit welcher der Behälter von außen deformiert ist.

Wenn gemäß einer weiteren Ausführungsform der Erfindung die Behandlungsvorrichtung mehrere Behälter jeweils mit einer Austauscheinrichtung und einer Zellexpositionseinrichtung, die biologisches Zellmaterial aufweist, umfasst, kann die Wirksamkeit der Steigerung der Immuntoleranz vorteilhafterweise erhöht werden. Besonders bevorzugt enthalten die Behälter verschiedene Typen biologischen Materials, so dass eine breitbandige Steigerung der Immuntoleranz, d. h. eine gleichzeitige Steigerung der Immuntoleranz in Bezug auf verschiedene Antigene erzielt wird.

Weitere Vorteile der Erfindung können sich ergeben, wenn die Behandlungsvorrichtung Wirkstoffe, welche die Entwicklung der Immuntoleranz fördern, insbesondere indem sie regulatorische T-Zellen fördern oder auf das mTOR-System wirken, und/oder mindestens eine gerinnungshemmende Substanz und/oder Wirkstoffe enthält, die eine immuntoleranzsteigernde Wirkung besitzen und/oder eine unterstützende Funktion bei der Induktion der Immuntoleranz ausüben. Es können beispielsweise Wirkstoffe vorgesehen sein, die eine Reaktion der von körpereigenen Immunzellen im Blut verstärken, die Verweildauer von spezifischen Blutbestandteilen verlängern, vorbestimmte Blutbestandteile anreichern, die Immuntoleranz verlängern, die Blutgerinnung hemmen, die Immuntoleranz verstärken und/oder die Immuntoleranz nach Rückgabe des modulierten Blutes vermitteln (nachgeschaltete Verstärkung.

Gemäß einer weiteren bevorzugten Variante der Erfindung kann die Behandlungsvorrichtung mit einer Messeinrichtung, die zur Erfassung von aus dem Blut an das biologische Zellmaterial gebundenen Substanzen, insbesondere Antikörpern, eingerichtet ist, ausgestattet sein. Vorteilhafterweise kann damit die Behandlungsvorrichtung zu analytischen oder diagnostischen Zwecken genutzt werden. Zum Beispiel kann die Menge an IgG-Antikörpern gemessen werden, die an das körperfremde oder körpereigene biologische Zellmaterial gebunden werden. Im Gegensatz zu üblichen immunologischen Tests (z.B. immunchromatographische Tests, ELISA) werden dabei nicht selektiv ausgewählte Antikörperbindungsstellen präsentiert, sondern eine Vielzahl möglicher Bindungsstellen. Dies ermöglicht breitgefächerte Analysen oder die frühe Detektion einer Autoimmunerkrankung.

Alternativ oder zusätzlich kann die Behandlungsvorrichtung mit einer Sammeleinrichtung, die zur Sammlung von aus der Blutprobe an das biologische Zellmaterial gebundenen Substanzen, insbesondere Antikörpern, eingerichtet ist, ausgestattet sein. Vorteilhafterweise kann damit die Behandlungsvorrichtung genutzt werden, um spezifische Selektionsprozesse im Empfängerblut durchzuführen. Hierbei werden entweder die an körperfremdes Zellmaterial gebundenen Bestandteile oder die im Blut enthaltenden nicht bindenden Bestandteile gesammelt und weiterverarbeitet.

Vorteilhafterweise gibt es verschiedene Varianten, die zu behandelnde Blutprobe in der Behandlungsvorrichtung bereitzustellen. Gemäß der Erfindung wird die zu behandelnde Blutprobe, insbesondere basierend auf einer Eigenblutspende des Probanden, in einem Zufuhrreservoir, insbesondere in einem Beutel, bereitgestellt und in ein Auffangreservoir, insbesondere in einen Beutel, ausgeleitet. Vorteilhafterweise erfolgt gemäß der Erfindung die Blutbehandlung, ohne dass der Proband anwesend ist. Beispielsweise kann bereits Wochen oder Monate vor einer geplanten Transplantation der Empfänger mehrfach Blut spenden, aus dem die Blutprobe für die erfindungsgemäße Behandlung gewonnen wird. Die Behandlung mit der Behandlungsvorrichtung erfolgt jeweils in einem Labor, einer Klinik/einem Behandlungszentrum, bis eine gewünschte Steigerung der Immuntoleranz erzielt ist. Zur Vermittlung der Immuntoleranz vor der Transplantation, wird dem Empfänger das behandelte Blut rückgeführt.

Alternativ kann die Behandlungsvorrichtung mit dem Blutkreislauf des Probanden verbunden werden, wobei das zu behandelnde Blut in die Behandlungsvorrichtung und von dieser zurück in den Blutkreislauf strömt (keine Ausführungsform der Erfindung). Die Verbindung mit dem Blutkreislauf des Probanden kann eine Verbindung mindestens einer Leitung der Austauscheinrichtung über eine Kanüle der jeweiligen Leitung direkt mit einem Blutgefäß und/oder einen Anschluss mindestens einer Leitung der Austauscheinrichtung an einem am Blutgefäß vorab vorhandenen Fluidanschluss, z. B. Kanüle, Katheter oder Port, umfassen.

Weitere Einzelheiten und Vorteile der Erfindung werden im Folgenden unter Bezug auf die beigefügten Zeichnungen beschrieben. Es zeigen:
- Figur 1: eine schematische Übersichtsdarstellung einer Ausführungsform der erfindungsgemäßen Behandlungsvorrichtung;
- Figuren 2 bis 4: Merkmale weiterer Ausführungsformen der erfindungsgemäßen Behandlungsvorrichtung;
- Figur 5: Merkmale eines Verfahrens zur extrakorporalen Blutbehandlung (keine Ausführungsform der Erfindung);
- Figur 6: Merkmale von Ausführungsformen des erfindungsgemäßen Verfahrens zur extrakorporalen Blutbehandlung; und
- Figur 7: eine schematische Übersichtsdarstellung von Merkmalen weiterer Ausführungsformen der erfindungsgemäßen Behandlungsvorrichtung.

Ausführungsformen der Erfindung werden im Folgenden in Bezug auf den Aufbau der Behandlungsvorrichtung und deren Anwendung beschrieben. Einzelheiten der Präparation des Zellmaterials, z. B. der Herstellung und ggf. Differenzierung von iPS-Zellen oder adulten Stammzellen, werden nicht beschrieben, da diese an sich bekannt sind. Es wird beispielhaft auf eine Behandlungsvorrichtung mit einem zylinderförmigen Behälter und einer einseitig an den Innenraum angrenzenden Zellexpositionseinrichtung Bezug genommen. Die Umsetzung der Erfindung ist nicht auf diese Ausführungsform beschränkt, sondern entsprechend mit anderen Behälterformen und anders konfigurierten Zellexpositionseinrichtungen möglich.

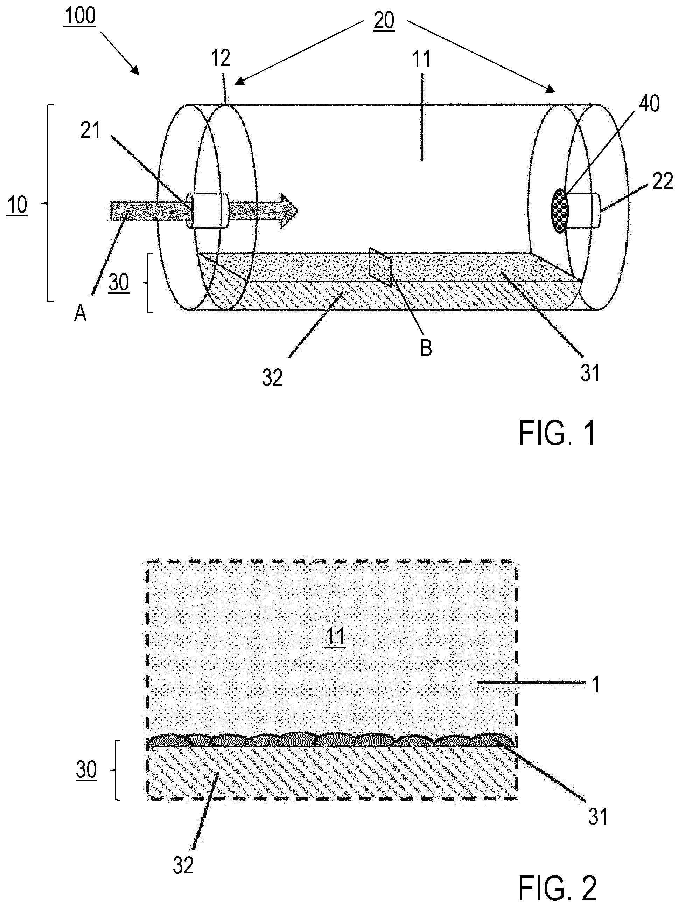

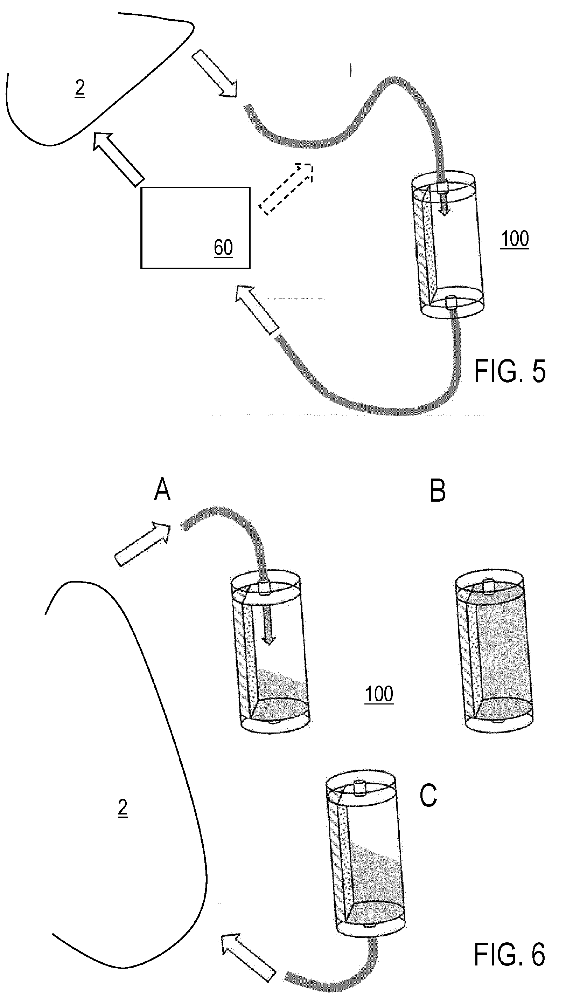

Figur 1 zeigt schematisch eine Ausführungsform der Behandlungsvorrichtung 100, umfassend einen zylinderförmigen Behälter 10, der einen Innenraum 11 zur Aufnahme einer Blutprobe 1 und eine Austauscheinrichtung 20 aufweist, eine Zellexpositionseinrichtung 30 mit biologischem Zellmaterial 31, und eine Rückhalteeinrichtung 40. Stirnseiten des Innenraums 11 sind mit sterilen Versiegelungswänden 12 verschlossen. Die Austauscheinrichtung 20 umfasst eine Eingangsleitung 21 und eine Ausgangsleitung 22 (von denen jeweils nur ein Abschnitt gezeigt ist, siehe auch Figuren 5 und 6), die an den gegenüber liegenden Stirnseiten des Innenraums 11 angeordnet sind und durch die Versiegelungswände 12 führen. Die Eingangsleitung und die Ausgangsleitung enthalten jeweils ein verschließbares Ventil (nicht gezeigt) zur Trennung des Innenraums 11 von der Umgebung, z. B. während der Behandlung der Blutprobe 1. Der Innenraum 11 hat ein Volumen von beispielsweise 100 ml.

Die Zellexpositionseinrichtung 30 umfasst das biologische Zellmaterial 31, das auf einem festen Träger 32 adhärent gebunden angeordnet ist. Das biologische Zellmaterial 31 umfasst z. B. bei der Behandlung der Blutprobe 1 vor einer Nierentransplantation differenzierte iPS-Zellen, die aus einem Spendergewebe hergestellt und zu Nierengewebe differenziert sind. Im dargestellten Beispiel hat das biologische Zellmaterial 31 eine freie Oberfläche, so dass die Blutprobe 1 im Innenraum 11 direkt das biologische Zellmaterial 31 berührt (siehe vergrößerter Ausschnitt B aus Figur 1 in Figur 2).

Die Rückhalteeinrichtung 40 umfasst einen Filter, der an der Ausgangsleitung 22 angebracht ist und chemisch und/oder mechanisch unbeabsichtigt frei gegebene Teile des Zellmaterials 31 in der Behandlungsvorrichtung 100 zurückhält.

Der Behälter 10 bildet ein Volumen, das über mindestens einen Zugang, wie die Eingangsleitung 11, mit der Blutprobe 1 des Probanden befüllt wird (siehe Pfeil A). Der Behälter 10 kann wie dargestellt zylindrisch, oder alternativ anders, z. B. quaderförmig oder prismenförmig, geformt sein. Nach der Inkubation der Blutprobe 1 in dem Innenraum 11 und in Anwesenheit des körperfremden Zellmaterials, z. B. für die Dauer von 4 Stunden, wird die Blutprobe 1 entweder über den zuvor benutzten Zugang oder einen zweiten Zugang, wie die Ausgangsleitung 12, entnommen. Alternativ zur Darstellung in Figur 1 ist es möglich, die Behandlungsvorrichtung 100 direkt an eine Kanüle anzuschließen und das Blut direkt über die Kanüle in den Behälter und wieder zurück in den Probanden zu überführen. Vorteilhafterweise wird damit die Kontaminationsgefahr gesenkt.

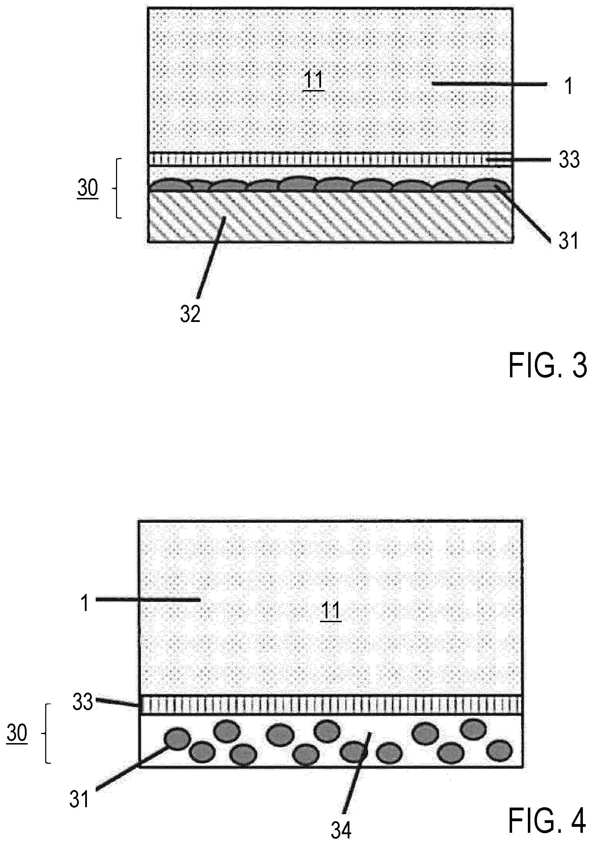

In dem Behälter 10 befindet sich auf dem Trägermaterial 32 das körperfremde biologische Zellmaterial 31, welches vorzugsweise Bindungsmöglichkeiten besitzt und diese den Immunsystembestandteilen (z.B. T- und B-Zellen) in der Blutprobe 1 präsentiert. Das körperfremde Zellmaterial 31 kann durch eine darüber liegende Trennschicht 33, wie z. B. eine Membran oder Gerüststruktur, mechanisch stabilisiert werden (siehe Figur 3). Das Trägermaterial 32 Polymere, Biopolymere und/oder Hydrogele umfassen. Des Weiteren können Gerinnungshemmer in das Trägermaterial 32 und/oder den Innenraum 11 eingebracht werden, um so die Gefahr einer Blutgerinnung zu vermindern. Des Weiteren können Wirkstoffe in dem Trägermaterial 32 und/oder dem Innenraum 11 angeordnet sein, welche die Entwicklung der Immuntoleranz fördern, wie z. B. der Wirkstoff Rapamycin (siehe [2]).

Da die Behandlungsvorrichtung 100 in medizinischen Institutionen (Arztpraxis, Krankenhaus, Labor) vor Ort eingesetzt werden soll, kann der Behälter 10 als Ganzes eingefroren (z. B. unter - 130 °C) und gelagert werden. Wenn die Behandlungsvorrichtung 100 dann eingesetzt werden soll, kann das Auftauen in einem bestimmten Zeitraum zuvor erfolgen, oder aber die Zellen werden direkt mit der warmen Blutprobe 1 des Probanden aufgetaut. Alternativ kann ausschließlich das Trägermaterial 32 mit dem körperfremden Zellmaterial 31 gesondert gefroren eingefroren gelagert werden. Hierbei wird das Trägermaterial 32 mit dem körperfremden Zellmaterial 31 kurz vor der Anwendung aufgetaut und in den Behälter 10 überführt oder direkt in dem Behälter 10 aufgetaut.

Die Figuren 3 und 4 zeigen alternative Gestaltungen der Zellexpositionseinrichtung 30 (entsprechend dem Ausschnitt B aus Figur 1). Bei diesen Ausführungsformen ist eine Trennschicht 33 vorgesehen, die das biologische Zellmaterial 31 vom Innenraum 11 trennt. Dabei kann das biologische Zellmaterial 31 fest auf einem Träger 32 fixiert (siehe Figur 3) oder trägerfrei in einem Suspensionsraum 34 suspendiert (siehe Figur 4) angeordnet sein. Die Trennschicht 33 ist für die Zellen des Zellmaterials 31 und deren Zellbestandteile, wie Zellmembranstücke, undurchlässig, jedoch für Antigen-Moleküle durchlässig. Die Trennschicht 33 umfasst z. B. Polymere und/oder Hydrogele.

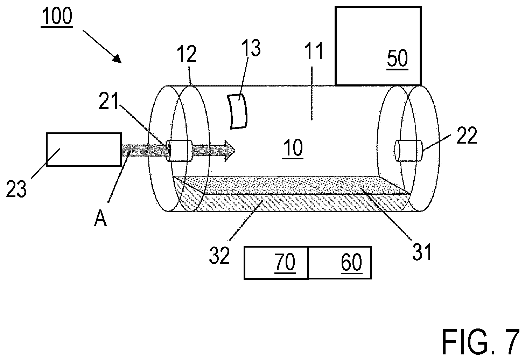

Figur 5 illustriert eine Verfahrensvariante, bei der die Behandlungsvorrichtung 100 durchgehender mit dem Blutkreislauf des Probanden 2 gekoppelt ist (keine Ausführungsform der Erfindung). Blut strömt, unter der Wirkung des Blutdrucks, ggf. unterstützt durch die Gravitation und/oder eine Pumpe (siehe Figur 7) direkt vom Probanden in die Behandlungsvorrichtung 100 und von dieser zurück zum Probanden 2. Dabei kann in der Ausgangsleitung eine Messeinrichtung 60 vorgesehen sein, mit der die erreichte Immuntoleranz im strömenden Blut quantitativ erfasst wird. Beispielsweise lassen sich gegen das körperfremde Zellmaterial gerichtete Antikörper (gegen Haupthistokompatibilitätskomplexe) im Blut des Probanden messen. Bei Stichprobenmessungen lässt sich z.B. eine EliSpot-Messung anwenden. In Abhängigkeit von dem Messergebnis kann eine erneute Rückführung des Bluts in die Behandlungsvorrichtung 100 vorgesehen sein (siehe gestrichelter Pfeil), um die Immuntoleranz weiter zu steigern.

Alternativ oder zusätzlich kann auch die Behandlungsvorrichtung 100, insbesondere das biologische Zellmaterial in dieser zur Evaluation der Immunreaktion genutzt werden. Hierbei werden die adhärenten Zellen in der Behandlungsvorrichtung 100 nach der Blutreaktion analysiert. Dazu kann bei einer Immunreaktion die verringerte Viabilität der adhärenten Zellen gemessen werden. Außerdem können an den Zellen gebundene Antikörper detektiert und quantifiziert werden (z.B. durch humane Antikörper, die gegen Tier-Antigene gerichtet sind.)

Figur 6 illustriert eine Ausführungsform des erfindungsgemäßen Verfahrens, bei dem in einem ersten Schritt (siehe Figur 6A) eine Beladung der Behandlungsvorrichtung 100 mit einer Blutprobe erfolgt. Die Blutprobe wird wie in Figur 5 direkt aus dem Probanden entnommen oder durch eine Blutspende in ein Zufuhrreservoir (nicht gezeigt) gefüllt und von dem Zufuhrreservoir in die Behandlungsvorrichtung 100 überführt. Anschließend erfolgt die Inkubation der Blutprobe (ohne Anschluss an den Probanden 2) in der Behandlungsvorrichtung 100 (siehe Figur 6B). Nach der Behandlung der Blutprobe erfolgt die Rückführung des Blutes in den Probanden 2 (siehe Figur 6C). Auch in diesem Fall kann eine Messung zur Evaluation der Immunreaktion am Blut und/oder Zellmaterial vorgesehen sein.

Figur 7 zeigt weitere Komponenten der Behandlungsvorrichtung 100, die einzeln oder in Kombination vorgesehen sein können. Die Behandlungsvorrichtung 100 ist wie in Figur 1 gezeigt. Zusätzlich ist eine Pumpe 23 vorgesehen, mit der der Blutdurchfluss durch den Behälter 10 unterstützt wird. Des Weiteren kann eine schematisch gezeigte, verschließbare Zugrifföffnung 13, zum Beispiel für Mess- und/oder Beobachtungszwecke, in einer Behälterwand vorgesehen sein.

Die schematisch gezeigte Konvektionseinrichtung 50 umfasst z. B. ein Schwenk- und/oder Deformationswerk, mit dem der Behälter 10 bewegt und/oder deformiert werden kann, um die Blutprobe 1 im Innerraum 11 des Behälters 10 zu bewegen. Alternativ oder zusätzlich kann im Innenraum ein Magnetrührer (nicht gezeigt) vorgesehen sein.

Des Weiteren zeigt Figur 7 schematisch die Messeinrichtung 60, die zur Erfassung von aus der Blutprobe 1 an das biologische Zellmaterial 31 gebundenen Substanzen, insbesondere Antikörpern, eingerichtet ist (siehe Figur 5), und eine Sammeleinrichtung 70, die zur Sammlung von aus der Blutprobe 1 an das biologische Zellmaterial 31 gebundenen Substanzen, insbesondere Antikörpern, oder die im Blut enthaltenen, nicht bindenden Bestandteile eingerichtet ist.

Die in der vorstehenden Beschreibung, den Zeichnungen und den Ansprüchen offenbarten Merkmale der Erfindung können sowohl einzeln als auch in Kombination oder Unterkombination für die Verwirklichung der Erfindung in ihren verschiedenen Ausgestaltungen von Bedeutung sein. Die Erfindung wird in den Ansprüchen definiert.

## Patentansprüche

1. Behandlungsvorrichtung (100), die zur extrakorporalen Blutbehandlung von Blut eines Probanden (2) für eine Immuntoleranz-Steigerung des Probanden (2) eingerichtet ist, umfassend
- eine Zellexpositionseinrichtung (30), die biologisches Zellmaterial (31) mit Oberflächenmolekülen aufweist, das für eine stoffliche Wechselwirkung mit der Blutprobe (1), umfassend eine Reaktion von körpereigenen Immunzellen im Blut mit den Oberflächenmolekülen des Zellmaterials (31), vorgesehen ist,
**gekennzeichnet durch**
- mindestens einen Behälter (10), der ein nach außen verschließbares Gefäß ist und einen Innenraum (11), in dem das biologische Zellmaterial (31) angeordnet ist, zur Aufnahme einer Blutprobe (1) vom Blut des Probanden (2) und eine Austauscheinrichtung (20) aufweist, die zur Zuleitung und/oder Ableitung der Blutprobe (1) in oder aus dem Innenraum (11) des Behälters (10) eingerichtet ist, wobei
- die Zellexpositionseinrichtung (30) im Behälter (10) positioniert ist, wobei
- das biologische Zellmaterial (31) zelläquivalente iPS-Zellen und/oder -Zellbestandteile, welche die gleichen Antigene tragen wie Zellen, aus denen die iPS-Zellen gewonnen wurden, und/ oder organäquivalent differenzierte iPS-Zellen und/oder -Zellbestandteile, welche die gleichen Antigene tragen wie Zellen der Organe, aus denen die iPS-Zellen gewonnen wurden, umfasst.

2. Behandlungsvorrichtung gemäß Anspruch 1, bei der
- die Zellexpositionseinrichtung (30) eine Trennschicht (33) aufweist, die eine teildurchlässige Membran oder ein teildurchlässiges Gitternetz umfasst, wobei mit der Trennschicht (33) das biologische Zellmaterial (31) vom Innenraum (11) getrennt ist und das biologische Zellmaterial (31) an der Zellexpositionseinrichtung (30) gehalten wird und die Trennschicht (33) für einen Substanzaustausch zwischen dem biologischen Zellmaterial (31) und der Blutprobe (1) eingerichtet ist.

3. Behandlungsvorrichtung gemäß einem der vorhergehenden Ansprüche, bei der
- die Zellexpositionseinrichtung (30) einen Träger (32) aufweist, an dem das biologische Zellmaterial (31) in einem adhärenten Zustand angeordnet ist.

4. Behandlungsvorrichtung gemäß Anspruch 3, bei der
- der Träger (32) eine Bindungsschicht trägt, die zur adhärenten Bindung von induzierten pluripotenten Stammzellen (iPS-Zellen), adulten Stammzellen und/oder daraus differenzierten Zellen eingerichtet ist.

5. Behandlungsvorrichtung gemäß einem der vorhergehenden Ansprüche, bei der
- die Zellexpositionseinrichtung (30) einen Suspensionsraum (34) aufweist, in dem das biologische Zellmaterial (31) in einem suspendierten Zustand und/oder auf Träger-Beads angeordnet ist.

6. Behandlungsvorrichtung gemäß einem der vorhergehenden Ansprüche, bei der die Austauscheinrichtung (20) mindestens eines der Merkmale umfasst
- die Austauscheinrichtung (20) weist eine Eingangsleitung (21) und eine Ausgangsleitung (22) auf, die in einer fluidischen Verbindung mit dem Innenraum (11) und mit einem gegenseitigen Abstand angeordnet sind,
- die Austauscheinrichtung (20) ist mit einer Kanüle ausgestattet, die zur direkten Verbindung mit dem Blutkreislauf des Probanden (2) eingerichtet ist, und
- die Austauscheinrichtung (20) ist mit einer Pumpe (23) ausgestattet, die zum Bluttransport in den oder aus dem Behälter (10) angeordnet ist.

7. Behandlungsvorrichtung gemäß einem der vorhergehenden Ansprüche, die umfasst
- eine Rückhalteeinrichtung (40), die für eine Zurückhaltung des biologischen Zellmaterials (31) im Behälter (10) angeordnet ist, wobei
- die Rückhalteeinrichtung (40) einen mechanisch oder chemisch wirksamen Filter umfasst und/oder ein Teil der Austauscheinrichtung (20) ist.

8. Behandlungsvorrichtung gemäß einem der vorhergehenden Ansprüche, die umfasst
- eine Konvektionseinrichtung (50), die zur Bewegung der Blutprobe (1) im Innerraum des Behälters (10) eingerichtet ist.

9. Behandlungsvorrichtung gemäß Anspruch 8, bei der
- die Konvektionseinrichtung (50) ein Rührwerk im Innenraum (11) und/oder ein Schwenkwerk umfasst, mit dem der Behälter (10) gekoppelt ist, und/oder
- der Behälter (10) eine flexible Behälterwand aufweist und die Konvektionseinrichtung (50) eine Deformationseinrichtung umfasst, mit welcher der Behälter (10) deformiert ist.

10. Behandlungsvorrichtung gemäß einem der vorhergehenden Ansprüche, umfassend
- mehrere Behälter (10) jeweils mit einer Austauscheinrichtung (20) und einer Zellexpositionseinrichtung (30), die biologisches Zellmaterial (31) aufweist, wobei
- die Behälter (10) verschiedene Typen biologischen Materials enthalten.

11. Behandlungsvorrichtung gemäß einem der vorhergehenden Ansprüche, die enthält
- Wirkstoffe, welche die Entwicklung der Immuntoleranz fördern, insbesondere indem sie regulatorische T-Zellen fördern oder auf das mTOR-System wirken,
- mindestens eine gerinnungshemmende Substanz, und/oder
- Wirkstoffe, die eine immuntoleranzsteigernde Wirkung besitzen und/oder eine unterstützende Funktion bei der Induktion der Immuntoleranz ausüben.

12. Behandlungsvorrichtung gemäß einem der vorhergehenden Ansprüche, die umfasst
- eine Messeinrichtung (60), die zur Erfassung von aus der Blutprobe (1) an das biologische Zellmaterial (31) gebundenen Substanzen, insbesondere Antikörpern, eingerichtet ist, und/oder
- eine Sammeleinrichtung (70), die zur Sammlung von aus der Blutprobe (1) an das biologische Zellmaterial (31) gebundenen Substanzen, insbesondere Antikörpern, eingerichtet ist.

13. Kit zur extrakorporalen Blutbehandlung, umfassend mindestens eine Behandlungsvorrichtung gemäß einem der vorhergehenden Ansprüche.

14. *In vitro* Verfahren zum Betreiben einer Behandlungsvorrichtung (100) gemäß einem der vorhergehenden Ansprüche, zur extrakorporalen Blutbehandlung von Blut eines Probanden (2) außerhalb des Körpers des Probanden (2) für eine Immuntoleranz-Steigerung des Probanden, umfassend die Schritte:
- Bereitstellen einer zu behandelnden Blutprobe (1) vom Blut des Probanden (2) in einem Zufuhrreservoir,
- Einleitung der Blutprobe (1) von dem Zufuhrreservoir in den Behälter (10) der Behandlungsvorrichtung (100),
- Behandlung der Blutprobe (1) durch eine Wechselwirkung, umfassend eine Reaktion der körpereigenen Immunzellen mit den Oberflächenmolekülen des präsentierten Zellmaterials (31) derart, dass die behandelte Blutprobe (1) geeignet ist, eine gesteigerte Immuntoleranz des Probanden (2) zu bewirken, und
- Ausleitung der Blutprobe (1) aus dem Behälter (10) in ein Auffangreservoir, wobei
- die Blutbehandlung erfolgt, ohne dass der Proband (2) anwesend ist.

15. Verfahren gemäß Anspruch 14, bei dem
- die Blutprobe (1) während der Behandlung in der Behandlungsvorrichtung (100) bewegt wird, und/oder
- eine Einstellung einer Menge und/oder Konzentration des biologischen Zellmaterials (31) in der Behandlungsvorrichtung (100) in Abhängigkeit von einer gegebenen oder erreichten Immuntoleranz des Probanden (2) vorgesehen ist.

16. Verfahren gemäß einem der Ansprüche 14 bis 15, bei dem
- die Zellexpositionseinrichtung (30) mit dem biologischen Zellmaterial (31), im eingefrorenen Zustand bereitgestellt und vor der Einleitung der Blutprobe in den Behälter (10) der Behandlungsvorrichtung (100) aufgetaut wird.

## Claims

1. Treatment apparatus (100), which is adapted for extracorporeal blood treatment of blood from a subject (2) for an immunotolerance enhancement of the subject (2), comprising
- a cell exposure device (30) which has biological cell material (31) with surface molecules, which cell material is provided for a material interaction with the blood sample (1), comprising a reaction of endogenous immune cells in the blood with the surface molecules of the cell material (31),
**characterised by**
- at least one container (10) which is a vessel capable of being closed to its surroundings and has an interior (11) wherein the biological cell material (31) is arranged, for receiving a blood sample (1) of blood from the subject (2), and an exchange device (20) which is designed to supply and/or discharge the blood sample (1) into or out of the interior (11) of the container (10), wherein
- the cell exposure device (30) is positioned in the container (10), wherein
- the biological cell material (31) comprises cell-equivalent iPS cells and/or cell components which carry the same antigens as cells from which the iPS cells were obtained, and/or organ-equivalent differentiated iPS cells and/or cell components which carry the same antigens as cells of the organs from which the iPS cells were obtained.

2. The treatment apparatus according to claim 1, wherein
- the cell exposure device (30) has a separating layer (33) comprising a semipermeable membrane or a semipermeable grid, wherein the biological cell material (31) is separated from the interior (11) by the separating layer (33) and the biological cell material (31) is held on the cell exposure device (30) and the separating layer (33) is designed for a substance exchange between the biological cell material (31) and the blood sample (1).

3. The treatment apparatus according to one of the foregoing claims, wherein
- the cell exposure device (30) has a support (32) on which the biological cell material (31) is arranged in an adherent state.

4. The treatment apparatus according to claim 3, wherein
- the support (32) has a binding layer which is designed for an adherent binding of induced pluripotent stem cells (iPS cells), adult stem cells and/or cells differentiated therefrom.

5. The treatment apparatus according to one of the foregoing claims, wherein
- the cell exposure device (30) has a suspension space (34) in which the biological cell material is arranged in a suspended state and/or on carrier beads.

6. The treatment apparatus according to one of the foregoing claims, wherein the exchange device (20) comprises at least one of the features:
- the exchange device (20) has an inlet line (21) and an outlet line (22) which are in fluidic connection with the interior (11) and are arranged mutually spaced apart,
- the exchange device (20) is provided with a cannula which is designed for direct connection to the circulation of the subject (2), and
- the exchange device (20) is provided with a pump (23) which is arranged for blood transport into or out of the container (10).

7. The treatment apparatus according to one of the foregoing claims, which comprises
- a retaining device (40) which is arranged for retaining the biological cell material (31) in the container (10), wherein
- the retaining device (40) has a mechanically- or chemically-acting filter, and/or is part of the exchange device (20).

8. The treatment apparatus according to one of the foregoing claims, which comprises
- a convection device (50) which is designed for moving the blood sample (1) in the interior of the container (10).

9. The treatment apparatus according to claim 8, wherein
- the convection device (50) comprises a stirring mechanism in the interior (11) and/or a tilting mechanism, to which the container (10) is coupled, and/or
- the container (10) has a flexible container wall and the convection device (50) comprises a deformation device by means of which the container (10) is deformed.

10. The treatment apparatus according to one of the foregoing claims, comprising
- a plurality of containers (10) each with an exchange device (20) and a cell exposure device (30), which has biological cell material (31), wherein
- the containers (10) contain different types of biological material.

11. The treatment apparatus according to one of the foregoing claims, which contains
- active substances which promote the development of immunotolerance, in particular by promoting regulatory T cells or acting on the mTOR system,
- at least one anticoagulant substance, and/or
- active substances which have an immunotolerance-enhancing action and/or have a supporting function in inducing immunotolerance.

12. The treatment apparatus according to one of the foregoing claims, which comprises
- a measuring device (60) which is designed to detect substances from the blood sample (1) which are bound to the biological cell material (31), in particular antibodies, and/or
- a collecting device (70) which is designed to collect substances from the blood sample (1) which are bound to the biological cell material (31), in particular antibodies.

13. Kit for extracorporeal blood treatment, comprising at least one treatment apparatus according to one of the foregoing claims.

14. *In vitro* method for operating a treatment apparatus (100) according to one of the foregoing claims for extracorporeal blood treatment of blood from a subject (2) outside the body of the subject (2) for an immunotolerance enhancement of the subject, comprising the steps of:
- providing a blood sample (1) to be treated of blood from the subject (2) in a supply reservoir,
- introducing the blood sample (1) from the supply reservoir into the container (10) of the treatment apparatus (100),
- treating the blood sample (1) by an interaction, comprising a reaction of the endogenous immune cells with the surface molecules of the presented cell material (31) such that the treated blood sample (1) is suitable for effecting an enhanced immunotolerance of the subject (2), and
- discharging the blood sample (1) from the container (10) into a collection reservoir, wherein
- the blood treatment is carried out in the absence of the subject (2).

15. The method according to claim 14, wherein
- the blood sample (1) is moved during the treatment in the treatment apparatus (100), and/or
- adjusting an amount and/or concentration of the biological cell material (31) in the treatment apparatus (100) depending on a given or achieved immunotolerance of the subject (2).

16. The method according to one of claims 14 to 15, wherein
- the cell exposure device (30) with the biological cell material (31) is provided in a frozen state and is thawed before the blood sample is introduced into the container (10) of the treatment apparatus (100)

## Revendications

1. Dispositif de traitement (100) configuré pour le traitement extracorporel du sang d'un sujet (2) en vue d'une augmentation de la tolérance immunitaire du sujet (2), comprenant :
- un appareil d'exposition cellulaire (30), qui comporte un matériel cellulaire biologique (31) présentant des molécules de surface, qui est prévu pour une interaction matérielle avec l'échantillon de sang (1), comprenant une réaction de cellules immunitaires autologues présentes dans le sang avec les molécules de surface du matériel cellulaire (31),
**caractérisé par**
- au moins un récipient (10), qui est un contenant pouvant être fermé vis-à-vis de l'extérieur et qui présente un espace intérieur (11), dans lequel le matériel cellulaire biologique (31) est disposé, destiné à recevoir un échantillon de sang (1) provenant du sang du sujet (2), ainsi qu'un appareil d'échange (20) configuré pour l'amenée et/ou l'évacuation de l'échantillon de sang (1) dans ou hors de l'espace intérieur (11) du récipient (10),
- l'appareil d'exposition cellulaire (30) étant positionné dans le récipient (10),
- le matériel cellulaire biologique (31) comprenant des cellules iPS équivalentes à des cellules et/ou des composants cellulaires qui portent les mêmes antigènes que les cellules à partir desquelles les cellules iPS ont été obtenues, et/ou des cellules iPS différenciées équivalentes à un organe et/ou des composants cellulaires qui portent les mêmes antigènes que les cellules des organes à partir desquels les cellules iPS ont été obtenues.

2. Dispositif de traitement selon la revendication 1, dans lequel
- l'appareil d'exposition cellulaire (30) comporte une couche de séparation (33), qui comprend une membrane semi-perméable ou un treillis semi-perméable, le matériel cellulaire biologique (31) étant séparé de l'espace intérieur (11) par la couche de séparation (33) et le matériel cellulaire biologique (31) étant maintenu sur l'appareil d'exposition cellulaire (30) et la couche de séparation (33) étant configurée pour permettre un échange de substances entre le matériel cellulaire biologique (31) et l'échantillon de sang (1).

3. Dispositif de traitement selon l'une quelconque des revendications précédentes, dans lequel
- l'appareil d'exposition cellulaire (30) comporte un support (32), sur lequel le matériel cellulaire biologique (31) est disposé dans un état adhérent.

4. Dispositif de traitement selon la revendication 3, dans lequel
- le support (32) porte une couche de liaison configurée pour assurer la liaison adhérente de cellules souches pluripotentes induites (cellules iPS), de cellules souches adultes et/ou de cellules différenciées dérivées de celles-ci.

5. Dispositif de traitement selon l'une quelconque des revendications précédentes, dans lequel
- l'appareil d'exposition cellulaire (30) comporte un espace de suspension (34), dans lequel le matériel cellulaire biologique (31) est disposé à l'état suspendu et/ou sur des billes porteuses.

6. Dispositif de traitement selon l'une quelconque des revendications précédentes, dans lequel l'appareil d'échange (20) présente au moins une des caractéristiques suivantes :
- l'appareil d'échange (20) comporte une conduite d'entrée (21) et une conduite de sortie (22), qui sont disposées en liaison fluidique avec l'espace intérieur (11) et à distance l'une de l'autre,
- l'appareil d'échange (20) est équipé d'une canule configurée pour être reliée directement à la circulation sanguine du sujet (2), et
- l'appareil d'échange (20) est équipé d'une pompe (23), qui est disposée pour transporter le sang dans le récipient (10) ou hors de celui-ci.

7. Dispositif de traitement selon l'une quelconque des revendications précédentes, comprenant :
- un appareil de retenue (40), qui est disposé pour retenir le matériel cellulaire biologique (31) dans le récipient (10),
- l'appareil de retenue (40) comprenant un filtre agissant mécaniquement ou chimiquement et/ou faisant partie de l'appareil d'échange (20).

8. Dispositif de traitement selon l'une quelconque des revendications précédentes, comprenant :
- un appareil de convection (50), qui est configuré pour mettre en mouvement l'échantillon de sang (1) dans l'espace intérieur du récipient (10).

9. Dispositif de traitement selon la revendication 8, dans lequel
- l'appareil de convection (50) comprenant un agitateur dans l'espace intérieur (11) et/ou un mécanisme de basculement auquel le récipient (10) est couplé, et/ou
- le récipient (10) présentant une paroi de récipient flexible, et l'appareil de convection (50) comprenant un appareil de déformation avec lequel le récipient (10) est déformé.

10. Dispositif de traitement selon l'une quelconque des revendications précédentes, comprenant :
- plusieurs récipients (10), chacun doté d'un appareil d'échange (20) et d'un appareil d'exposition cellulaire (30) comportant un matériel cellulaire biologique (31),
- les récipients (10) contiennent différents types de matériel biologique.

11. Dispositif de traitement selon l'une quelconque des revendications précédentes, contenant :
- des substances actives qui favorisent le développement de la tolérance immunitaire, notamment en favorisant les cellules T régulatrices ou en agissant sur le système mTOR,
- au moins une substance anticoagulante, et/ou
- des substances actives qui possèdent un effet augmentant la tolérance immunitaire et/ou exercent une fonction de soutien lors de l'induction de la tolérance immunitaire.

12. Dispositif de traitement selon l'une quelconque des revendications précédentes, comprenant :
- un appareil de mesure (60), qui est configuré pour détecter des substances, notamment des anticorps, liées à partir de l'échantillon de sang (1) au matériel cellulaire biologique (31), et/ou
- un appareil de collecte (70), qui est configuré pour collecter des substances, notamment des anticorps, liées à partir de l'échantillon de sang (1) au matériel cellulaire biologique (31).

13. Kit de traitement extracorporel du sang, comprenant au moins un dispositif de traitement selon l'une quelconque des revendications précédentes.

14. Procédé in vitro d'exploitation d'un dispositif de traitement (100) selon l'une quelconque des revendications précédentes, destiné au traitement extracorporel du sang d'un sujet (2) à l'extérieur du corps du sujet (2) en vue d'une augmentation de la tolérance immunitaire du sujet, comprenant les étapes suivantes :
- la fourniture d'un échantillon de sang (1) à traiter provenant du sang du sujet (2) dans un réservoir d'alimentation,
- l'introduction de l'échantillon de sang (1) depuis le réservoir d'alimentation dans le récipient (10) du dispositif de traitement (100),
- le traitement de l'échantillon de sang (1) par une interaction comprenant une réaction des cellules immunitaires autologues avec les molécules de surface du matériel cellulaire présenté (31), de telle sorte que l'échantillon de sang traité (1) soit apte à provoquer une augmentation de la tolérance immunitaire du sujet (2), et
- l'évacuation de l'échantillon de sang (1) du récipient (10) vers un réservoir de collecte,
- le traitement du sang est effectué sans que le sujet (2) soit présent.

15. Procédé selon la revendication 14, dans lequel
- l'échantillon de sang (1) est mis en mouvement pendant le traitement dans le dispositif de traitement (100), et/ou
- un réglage de la quantité et/ou de la concentration du matériel cellulaire biologique (31) dans le dispositif de traitement (100) est prévu en fonction d'une tolérance immunitaire donnée ou atteinte du sujet (2).

16. Procédé selon l'une quelconque des revendications 14 à 15, dans lequel
- l'appareil d'exposition cellulaire (30) contenant le matériel cellulaire biologique (31) est fourni à l'état congelé et est décongelé avant l'introduction de l'échantillon de sang dans le récipient (10) du dispositif de traitement (100).
